# EUROPEAN PATENT SPECIFICATION

(11) **EP 1 608 331 B1**
(45) Date of publication and mention of the grant of the patent: **07.11.2007**
(21) Application number: 04820793.0
(22) Date of filing: 18.03.2004
(51) Int. Cl.: A61K 8/25, A61K 8/37, A61K 8/35, A61Q 17/04

(54) **SUNSCREEN COMPOSITION COMPRISING A MIXTURE OF SILICAS**
SONNENSCHUTZMITTEL MIT EINEM SILIKA-GEMISCH
COMPOSITION D'ECRAN SOLAIRE RENFERMANT UN MELANGE DE SILICES

(30) Priority: 18.03.2003 BR 0300699
(43) Date of publication of application: 28.12.2005
(73) Proprietor: Johnson & Johnson Industrial Ltda., 12237-350 Sao José dos Campos SP (BR)
(72) Inventor: CONSUL DE MORAES, Alice Aparecida, Sao José dos Campos, SP (BR); RIBEIRO, Maycon, Sao José dos Campos, SP (BR)
(74) Representative: James, Anthony Christopher W.P.
(86) International application number: PCT/BR2004/000035
(87) International publication number: WO 2005/063190

(56) References cited:
- EP-A- 0 288 419
- EP-A- 0 919 222
- EP-A- 0 930 063
- US-A- 4 749 569
- US-A- 5 093 107
- US-A- 5 204 398
- US-A- 5 320 834
- S. HASENZAHL, A. BRAUNAGEL: "FUMED SILICA FOR PERSONAL CARE AND COSMETICS" SÖFW-JOURNAL, vol. 129, August 2003 (2003-08), XP002292235

## Description

### Field of the Art

Prolonged exposure of the human skin to ultraviolet (UV) radiation, which reaches the surface of the earth, may lead to various skin disorders, such as erythemas, dermatoses, dermatitises, skin cancers and accelerated aging of the skin.

Ultraviolet (UV) rays coming from the sun penetrate the skin and are absorbed by the epidermis, by the dermis and by the subcutaneous tissue. Under both sunlight and artificial tanning devices, the overexposure to ultraviolet rays generates effects that are harmful to the human skin. The skin also contains a pigment called melanin, which contributes to the protection of the epidermis cells, against solar radiation. UV radiation causes the melanin to grow dark, and this is visible arter a few days of sun-tanning. In addition, when the skin is exposed to sunlight, the so-called free radicals are released and cause damage to directly the skin tissue. With a prolonged exposure to sunlight, large amounts of free radicals are released and neutralized by the natural antioxidants of the organism, which decreases the reserves of natural antioxidants of the skin. The accumulation of free radicals damages the skin fiber, which causes wrinkling, parching and possibly cancer. Melanoma is certainly the type of cancer most harmful to the skin and is produced by transformation of the melacocyte.

As time goes by, the human capability of producing antioxidants decreases. This causes the malignant manifestations of the skin to appear usually in adulthood, as a result of predisposition and overexposure to the rays of the sun. This means that the damage produced by sunrays do not stop existing, even after the disappearance of their most evident effects (such as sunburns), since these effects progressively accumulate year after year. One may say that skin cancer is the extreme manifestation of the effects of UV bands. The World Health Organization (WHO) estimates that over two million cases of skin cancer in the world occur each year, 200,000 of which correspond to malignant-cancer melanomas. The excess sunlight also weakens the immune system, possibly increasing the risk of contracting infectious diseases.

Consciousness of the risks to health due to excess exposition to UV radiation has increased over the years, and the use of sunscreens has become quite predominant. Several sun-screen compositions have been developed, and such compositions absorb, disperse and block UV radiation, thus inhibiting the undesirable effects on the skin. Typical compositions are found in the form of sun lotions, creams, salves and gels, which considerably vary in their skin-protecting power.

Different types of sunscreen have been obtained, and their effectiveness has considerably evolved. The existing sunscreens are intended for protecting the user against sunburn during a limited exposure to sunlight. At present, due to the discovery that any extent of unprotected exposure may potentially cause suppression of the immune system and may lead to future health problems (such as skin narcinomas and other skin disturbances and/or problems), the pretension is to eliminate exposure to UV radiation as much as possible. Sun-tanning, rather than being strictly agreeable, is currently viewed by the medical community as a symptom of a damage to the skin when there is overexposure to UV radiation.

The international classification system FPS (Fator de Proteção Solar) or Sunlight Protection Factor (SPF) has been developed for the purpose of guiding the consumer in selecting sunscreens. In general, the SPF corresponds to about a multiple time during which the sunscreen will prevent redness of the skin, on the time of exposure that causes the unprotected skin to exhibit redness. Thus, if a sunscreen composition with a SPF of 10 is properly applied to an individual, he will be able to remain under sunlight without suffering visible effects ten times as long as the usual unprotected duration. The exposure necessary to produce a visible effect varies from person to person, due to the differences in their skin cells.

A sunscreen is generally used during outdoor activities. Many people have occupations that require exposure to sunlight for long periods of time. Also, there are those who spend their leisure time in amusement activities outdoors, such as, for example, sunbathing, surfing, fishing and swimming. All these activities promote perspiration/sweat/transpiration and/or allow the body to get into contact with different liquids, such as pure water, aqueous saline solution (seawater), aqueous chlorinated solution (swimming-pool water), aqueous solution with a high metallic degree (river water) and mixtures thereof. Said exposure to sunlight requires the use of sunscreens, but a number of them have limited effectiveness, since the protecting film around the skin is removed when in contact with said liquids, which allows direct attack of UV rays on the skin. Hence the need for the sunscreen to be resistant to such liquids, so it will not diminish its effectiveness.

There are a number of sunscreen compositions that absorb the ultraviolet radiation. These compositions are also resistant to skin removal by perspiration/sweat/transpiration or by external washing of the skin to prolong its effectiveness. Most of these compositions employ polymeric materials, which are emulsified or carried to the skin by a carrier in which a continuous polymeric film is molded around the skin.

Among the types of ultraviolet radiation, there are the emissions of UVA and UVB. The UVA emission is of long length and accounts for tanning and early aging of the skin. On the other hand, UVB emission has a shorter length and reaches more superficial layers of the skin, accounting for sunburns and skin cancer. One type of UV ray potentializes the other. The ideal thing is that sunscreens could combat both UVA and UVB emission.

At present, there is a growing consciousness of the need for sunlight protection on the part of the consumer, but even so the wish to get a suntan is very evident.

### Prior Art

Reference examples of the prior art related to this matter are presented below.

US Pat. 3,821,363 of Black and Feinstone describes the use of an acidic formulation of a cross-linked maleic anhydride copolymer in preparing a sunscreen gel.

US Pat. 3,895,104 of Karg discloses compositions and methods in which a resinous polyamide material is used as a sunscreen film.

US Pat. 4,172,122 of Minnesota Mining and Manufacturing Company defines the use of acidic compositions of acrylate/acrylic copolymer in formulating oils and emulsions for sunscreens.

US Pat. 4,193,989 of Anheuser-Busch Incorporated describes a waterproof sunscreen in-the form of a gel. This sunscreen consists of a sun-screen agent, an alcoholic solvent, a jellifying agent, an emollient, an acid and a hydroxypropyl cellulose polymer.

US Pat. 4,254,102 of Plough, Inc., discloses the use of sun-screen compositions containing hydroxyethyl cellulose polymeric agents in combination with a surfactant and a fatty alcohol.

US 4,710,371 of Palinezar discloses waterproof sunscreen compositions which mainly comprises butene isobutylene copolymers and UV-absorbing agents.

US Pat. 5,914,102 of Schering-Plough HealthCare discloses oil-based sunscreens having a high protection factor and resistance to transpiration. These sunscreens comprise formulations in the form of opaque oil-in-water emulsions, which comprise an emulsifier, an aqueous phase and an oily phase comprising hydrophobically treated silica particles and a UV-emission absorbing organic compound.

Patent application PCT/AU00/00814 (publication WO 01/03663 A1) discloses alcohol-based formulations in the form of a spray or a gel, comprising fumigated silica, volatile silicone, sunscreen agents and alcohol-based carriers.

The compositions known from the prior art, which employ polymers to give form to a continuous polymeric film, have a number of drawbacks. One of them is the high production cost. Water-based or alcohol-based compositions have low viscosity and are difficult to apply evenly onto the skin, thus allowing the occurrence of burn points on the skin, which may result in the referred-to disturbances and/or diseases of the skin.

Water-based compositions in which the polymer is present in an emulsion, usually have drawbacks; for instance, they take long to dry on the skin, foaming occurs during the application and there is a feeling of clamminess on the skin. If these compositions are not completely dried, they further present a trend to allow particulate material such as beach sand to adhere to the skin. Moreover, the water resistance property of water-based compositions is extremely reduced is they are not completely dried before perspiration/sweat/transpiration or direct contact with liquids.

The formation of a continuous sunscreen film on the skin is prevented by compositions that employ solvent systems, since they do not tolerate a large amount of oil or other emollients. Without the use of emollient in alcohol-based compositions the skin may become dry and irritated. In general, these compositions are also formulated in thin solutions with low viscosity, which render them difficult to apply evenly onto the skin.

US Pat. 4,710,370 discloses waterproof sunscreen compositions that essentially comprise butene isobutylene copolymers and UV-radiation absorbing agents (sunscreens). These compositions are based on oil - the use of mineral oil is mentioned - and, additionally, comprise other thickening agents, which are, among others, inorganic salts that may be fumigated and amorphous silicas, sodium silicate magnesium and colloidal magnesium silicate aluminum. As already mentioned, the use of a polymeric material significantly makes the production cost of these sunscreens expensive. Further, the end product available to the consumer of these compositions presents too much oiliness, which generates clamminess and a heavy and unpleasant touch.

### Summary of the Invention

The present invention relates to novel oil-based compositions for skin protection, which, when applied to the human skin, supply protection against the harmful effects caused by the emission of UV radiation from the sun and artificial tanning apparatus. Particularly, the present invention combines said protection against damages caused by exposure to emissions of UV with a healthful tanning of the skin.

More particularly, the present invention relates to novel oil-based compositions for skin protection, which considerably diminishes the damages caused by exposure to sunlight and bring about a suntan.

More particularly, the present invention discloses novel oily sunscreens, which provides a suntan and are resistant to liquids.

Still more particularly, the present invention discloses novel oily, liquid-resistant, sun-tanning sunscreen compositions in the form of transparent gels.

Once the sunscreen of the present invention has been spread on the skin, it remains thereon as a continuous even film adhered to the epithelium surface. Said film resists various liquids (pure water, aqueous saline solution, aqueous chlorinated solution, aqueous solution with a high metallic degree, mixtures thereof, among other liquids), be it by external washing of the skin or by perspiration/sweat/transpiration. Besides providing protection against emissions of UV, the composition of the present invention also promotes a healthful suntan of the skin.

The present invention solves the various problems and drawbacks of the prior art, which are identified along the present specification. As a non-limiting example, some improvements provided by the composition of the present invention in comparison with the prior art are listed below.
a) great reduction in the manufacture costs;
b) high degree of transparency of the end product;
c) rapid absorption;
d) dry, soft, light and non-clammy touch; and
e) useful and efficient combination of a sunscreen with a suntan of the skin, so as to provide more protection to the user.

Most sunscreen compositions of the prior art comprise a polymer or a mixture of polymers as thickening agents. Besides representing an expensive raw material, polymer interferes with the touch and transparency of the end product. As an example, the compositions of the prior art usually employ polyisobutene (heavier touch to the end product) and polyethylene (causes darkening of the end product). These compositions further comprise thickeners that do not thicken any type of carrier, as for example alkylgalactoma, which does not thicken any type of emollient carrier.

There are few sunscreens in the form of gel. Sunscreens are usually found in the form of creams and salves, which impart undesirable characteristics to the user, such as a heavier touch and more oiliness. Still fewer sunscreens are formulated in combination with suntan lotions.

The present invention discloses a transparent oil-based sun-screen, which is resistant to liquids and has not polymeric material as a thickening agent. In addition, another relevant characteristic of the present invention is a combination of said sunscreen with a sun-tanning agent. Cosmetic formulating agents such as, for example, fragrance agents, coloring agents, antibacterial agents, insect-repelling agents and vitamins, among others, can also be incorporated into the compositions of the present invention.

Particularly, the novel compositions of the present invention are formulated in the form of a transparent gel, providing a pleasant, soft and dry touch, rapid absorption and a refreshing feeling.

The composition of the invention has as its main characteristic the replacement of polymeric thickening material with a particular mixture of silicas as a thickening agent. The inventors have found an new combination of hydrophilic and hydrophobic silicas which are effective for association with sunscreens to provide sunscreen compositions, especially in the form of a transparent gel.

According to the present invention, the variation in the concentration of the hydrophilic and hydrophobic silicas enables the novel composition of the present invention to achieve the benefits described herein. Further, said variation in the concentrations of silicas prevents syneresis (separation of gel-oil phases) of the composition, which is undesirable when the latter is stored.

Hydrophilic materials are those that have more affinity with substances that are more polar. On the other hand, hydrophobic materials are those that have affinity with less polar liquid.

Another embodiment of the present invention is the use of a mixture of silicas for the preparation of the composition, which is also an object of the present invention.

A further embodiment of the present invention is the use of the composition for preparing an oil-based sunscreen, which may additionally be a suntan agent.

A further embodiment of the present invention is a method of preventing diseases of the skin, wherein the composition of the present invention is used.

### Detailed Description

The present invention discloses a novel composition for use as sunscreen against UV radiation, which may come either from the sun or from artificial tanning devices, which essentially comprises a UV-radiation emission filtering agent and a particular combination of silicas as a thickening agent.

The compositions of the invention may further comprise cosmetic formulation agents that are usually employed in the formulation of such compositions. Among others, the compositions may contain fragrance agents, coloring agents, antibacterial agents, insect-repelling agents and vitamins, mixtures thereof and the like.

The compositions of the present invention further comprise a carrier. The carriers employed in such compositions can be, for example: isopropyl myristate, isopropyl palmitate, alkyl benzoate, preferably mineral oil.

The carrier employed in the present invention is the mineral oil qsp. (for exmaple, MINERAL OIL).

The mineral oil basically constitutes the oily phase of various emulsions. It is a mixture of liquid hydrocarbons obtained from petroleum and is oily, colorless and transparent. It is tasteless and odorless when cold, with a slight smell of petroleum when heated. It has an emollient action and is useful in several conditions of skin irritation and for removing scabs.

The UV-radiation filtering agent is selected from the group consisting of ethylexyl metoxycinamate (for example, UVINUL MC 80), benzophenone-3 (oxybenzone), aminobenzoic acid, avobenzone, phenylbenzimidazol sulphonic acid, sulisobenzone, cynoxate, digaloyl trioleate, dietanolamine methoxycinamate, dioxybenzone, ethyl 4-[bis(hydroxypropyl)]aminobenzoate, 2-ethyl-hexyl 2-cyano-3,3-diphenylacrylate, homosalate, glyceryl aminobenzoate, methyl antranylate, octocrylene, ethylhexyl salicylate (for example, ETHYLHEXYL SALICYLATE) padimate A, padimate O and tinosorb S. Preferably, ethylhexyl metoxycinamate, oxybenzone and ethylhexyl salicylate.

The proportion of said chemical filters in the compositions is of about 0.1% to about 25% by weight. Preferably, the compositions of the present invention comprise from about 0.1% to about 10.0% of oxybenzone, about 0.5% to about 10% of ethylhexyl metoxycinamate and about .01% to about 5.0% of ethylhexyl salicilate. Still more preferably, the compositions of the present invention comprise about 0.3% to about 4.0% of oxybenzone, about 1.0% to about 7.0% of ethylhexyl metoxycinamate and about 0.5% to about 3.0% of ethylhexyl salicylate.

The thickener used in the compositions of the present invention is a mixture of hydrophobic and hydrophilic silicas, the total proportion of which in the compositions is of 1.0% to about 15.0% by weight, preferably about 2.0% to about 8.0% by weight, more preferably about 3.0% to about 7.0% by weight.

The silicas of the mixture of silicas represent about 0.85% to about 7.5% by weight of a more hydrophobic silica and about 0.1% to about 7.5% by weight of a more hydrophilic silica. Preferably, the silicas present in said mixture of silicas represent about 0.9% to about 7.0% by weight of more hydrophobic silica and about 0.15% to about 3.0% by weight of a more hydrophilic silica. Still more preferably, the silicas present in said mixture of silicas represent about 1.7% to about 5.3% by weight of a more hydrophobic silica and about 0.2% to about 1.7% by weight of a more hydrophilic silica. The proportion of hydrophilic silica to the hydrophobic silica may vary depending upon the specific type of oil used in the formulation.

The silicas that comprise such a mixture are, either separately or in combination, fumigated silica, amorphous silicas, molten silicas, vitreous silicas, colloidal silicas, silicate salts and/or mixtures thereof. An example of hydrophobic silica is Aerosil R from Degussa. An example of preferred hydrophobic silica is HDK H18 or H20 from Wacker (silica dimethyl sililate) An example of hydrophobic silica is Aerosil 200 from Degussa. An example of preferred hydrophilic silica is HDK N20 from Wacker (silica dimethyl sililate).

In the cosmetic area, it is widely known that silica may be a viscosity controller, a thickening agent and/or a thixotropic agent. The thickening and thixotropic effect of the silica will depend on the nature of the liquid, the concentration of the silica, the types of the other additives and the degree of dispersion. Specifically, non-polar liquids (for example, benzene or mineral oil) require small amounts of colloidal silica for producing an increase in viscosity when compared with polar liquids (for example, water or alcohol). In all the types of liquids, the amount of silica required may be reduced by adding determined additives. One usually consults with the manufacturer of silica for a more detailed description of: these phenomena. The end factor is the degree of dispersion. In non-polar liquids, the viscosity increases continuously with growing shearing and/frictions. In moderately polar liquids, the viscosity increases up to a peak and then drops down to plateau, while the shear ing/frictions increase. In polar liquids, the viscosity decreases until it reaches a plateau (while the shearing/frictions are increased). Properties such as specific surface and high internal porosity of the silicas impart an excellent result to them as a thickening agent. They are efficient in the formation of gels, pastes or creams for any desired viscosity, by adding a small amount thereof.

Although the inventors do not wish to be limited to specific theories, it is believed that the variation of hydrophobicity / hydrophilicity of the respective silicas present in the compositions of the present invention prevents separation of phases that would probably be expected if such components were combined at random with the oil phase of the compositions of the invention. The different properties of hydrophobicity / hydrophilicity of the silicas present in the mixture of silicas cooperate and interact with the respective properties inherent in the oils used in the formulation, thus ensuring non-separation of phases that could occur and providing a composition of a composition in the form of a gel that is transparent and of soft touch when applied. Similarly, the variations in concentrations of silicas in the mixture of silicas and in the end formulation are also relevant to achieve the desired results.

The formulation auxiliaries mentioned before may be selected, among others, from the group comprising:
- coloring agents: B-carotene, carrot oil (for example CARROT OIL)
- fragrance agents: (for example, FRAG. H&r 40205);
- antibacterial agents: propyl parabene, IPBC;
- insect-repelling agents; and
- vitamin agents: vitamin-A palmitate, vitamin-E acetate (for example, VIT. E ACETATE).

The compositions of the present invention preferably comprise about 0.1% to 1% by weight of fragrances, about 0.001% to 5% by weight of vitamins and about 0.001 to 1% by weight of antioxidants.

A highly preferred formulation according to the present invention basically comprises the following components:
- vitamin-E acetate;
- benzophenone 3;
- buthylhydroxytoluene;
- silica dimethyl sililate;
- fragrance;
- ethylhexyl metoxycinamate;
- carrot oil;
- mineral oil;
- ethylhexyl salicilate.

The novel compositions of the present invention are formulated in accordance with existing techniques, so that the end product is in the form of a gel, liquid, spray or roll-on. For the purpose of illustrating the process used in preparing the disclosed compositions, this process basically com-mixture of silicas, and then adding the silicas, by means of a homogenizer, until dispersion is reached.

Below, a few examples illustrate compositions embraced by the present invention, as well as the respective results achieved with the novel formulations described herein. The examples listed are merely intended for showing and illustrating the embodiment of the invention and not intended to limit it.

### Examples

| Function | Name INCl | Example 1 |
|---|---|---|
| Sunscreen | Benzophenone-3 | 1.50 |
| Sunscreen | Ethylhexyl methoxycinamate | 6.00 |
| Antioxidant | BHT | 0.05 |
| Vitamin | Vitamin-e acetate | 0.10 |
| Emollient | Carrot oil | 1.00 |
| Sunscreen | Ethylhexyl salicilate | 1.00 |
| Fragrance | Fragrance | 0.20 |
| Thickening agent | Silica dimethyl sililate | 1.0 |
| Carrier | Mineral oil | |
| | | 100.00 |

Or

| Function | Name INCl | Example 2 |
|---|---|---|
| Sunscreen | Benzophenone-3 | 1.00 |
| Sunscreen | Ethylhexyl methoxycinamate | 5.00 |
| Antioxidant | BHT | 0.05 |
| Antioxidant | Tocopherol acetate | 0.10 |
| Emollient | Carrot oil | 1.00 |
| Sunscreen | Ethylhexyl salicilate | 1.00 |
| Fragrance | Fragrance | 0.20 |
| Thickening agent | Silica dimethyl silicilate | 2.80 |
| Carrier | Ester | 88.85 |
| | | 100.00 |

As already approached before, from the above Examples we have novel compositions, formulated in the form of a transparent gel, which provide a pleasant, soft and dry touch, rapid absorption and a refreshing feeling.

Finally, the novel compositions defined herein are useful in preventing diseases of the skin, such as bums, erythemas, dermatosis, dermatitis and others. By applying onto the skin a prophylactically effective amount of the compositions of the present invention, before and/or during exposure of the skin to sunlight and/or to the artificial tanning system, one can prevent or even avoid the occurrence of the referred-to disturbances/diseases of the skin.

## Claims

1. A composition **characterized by** comprising:
a) a carrier;
b) a UV-radiation emission filtering agent;
c) a hydrophobic silica; and
d) a hydrophilic silica, wherein said composition is in the form of a liquid, gel, spray or roll-on, and wherein the composition is oil-based.

2. A composition according to claim 1, **characterized by** further comprising cosmetic formulation agents such as fragrance agents, coloring agents, antibacterial agents, insect-repelling agents and vitamins.

3. A composition according to claim 2, **characterized in that** the coloring agent is carrot oil.

4. A composition according to any one of claims 1 to 3, **characterized in that** said UV-radiation emission filtering agent is selected from a group consisting of ethylhexyl methoxycinamate, benzophenone-3, aminobenzoic acid, avobenzone, phenylbenzimidazol sulphonic acid, sulisobenzone, cinoxato, digaloyl trioleate, diethanolamine methoxycinamate, dioxybenzone, ethyl 4-[bis(hydroxypropyl)]aminobenzoate, 2-ethylhexyl 2-cyano-3,3-diphenylacrylate, homosalate, glyceryl aminobenzoate, methyl antranylate, octocrylene, ethylhexyl salicilate, padimate A, padimate O and Tinosorb S.

5. A composition according to any of the preceding claims, **characterized in that** the silicas present in said mixture of silicas represent about 0.85% to about 7.5% by weight of a more hydrophobic silica and about 0.1% to about 7.5% by weight of a more hydrophilic silica.

6. A composition according to claim 5, **characterized in that** the silicas present in said mixture of silicas represent about 0.9% to about 7.0% by weight of a more hydrophobic silica and about 0.15% to about 3.0% by weight of a more hydrophilic silica.

7. A composition according to claim 6, **characterized in that** the silicas present in said mixture of silicas represent about 1.7 to about 5.3% by weight of a more hydrophobic silica and about 0.2% to about 1.7% by weight of a more hydrophilic silica.

8. A composition according to any one of claims 1 to 7, **characterized in that** said mixture of silicas comprise, either separately or in combination, fumigated silica, amorphous silica, molten silica, vitreous silica, colloidal silica, silicate salts, silica dimethyl sililate and/or mixtures thereof.

9. A composition according to any one of claims 1 to 8, **characterized in that** said carrier is a mineral oil.

10. A composition according to any one of claims 1 to 9, **characterized in that** the components of the formulation are present in the following amounts :
a) about 0.1% to about 25% by weight of a UV-radiation filtering agent;
b) about 1.0% to about 15.0% by weight of a mixture of silicas;
c) about 0.001 to 1% by weight of antioxidants;
d) about 0.001 to about 5% by weight of vitamins; and
e) about 0.1% to about 1% by weight of fragrances.

11. Use of a mixture of hydrophobic and hydrophilic silicas, **characterized in that** it is intended for preparing a composition as defined in any one of claims 1 to 10.

12. Use according to claim 11, **characterized in that** said mixture of silicas has the following proportion:
i) about 0.85% to 7.5% by weight of a more hydrophobic silica; and
ii) about 0.1% to 7.5% by weight of a more hydrophilic silica; more preferably:
i) 0.9% by weight of a more hydrophobic silica; and
ii) about 0.15% to 3.0% by weight of a more hydrophilic silica; and more preferably:
i) about 1.7 to 5. 3% by weight of a more hydrophobic silica; and
ii) about 0.2% to 1.7% by weight of a more hydrophilic silica.

13. Use according to claim 11 or 12, **characterized in that** said mixture of silicas comprises, either separately or in combination, fumigated silica, amorphous silica, molten silica, vitreous silica, colloidal silica, silicate salts, silica dimethyl sililate and/or mixtures thereof.

14. Use of a composition as defined in any one of claims 1 to 10, **characterized in that** it is intended for the preparation of a cosmetic product.

15. Use according to claim 14, **characterized in that** said cosmetic product is a sunscreen or a tanning sunscreen.

16. Use according to claim 14 or 15, **characterized in that** said cosmetic product is a tanning sunscreen in transparent oily gel.

17. Use of a composition comprising:
a) carrier,
b) a UV-radiation filtering agent;
c) a hydrophobic silica; and
d) a hydrophilic silica
in the manufacture of a medicament for preventing diseases of the skin wherein the medicament is to be applied onto the skin of an individual.

18. A use according to claim 17, **characterized in that** said diseases of the skin are disturbances caused by exposure of the skin of an individual to emissions of UV radiation, which are selected from a group consisting of bum, erythemas, dermatoses, dermatitises, skin cancers and others.

## Patentansprüche

1. Zusammensetzung, **dadurch gekennzeichnet, daß** sie umfaßt:
a) einen Träger;
b) ein UV-Strahlungsemissions-Filtermittel;
c) ein hydrophobes Silica; und
d) ein hydrophiles Silica, wobei die Zusammensetzung die Form einer Flüssigkeit, eines Gels, eines Sprays oder eines Roll-on aufweist und wobei die Zusammensetzung eine Ölbasis besitzt.

2. Zusammensetzung nach Anspruch 1, **dadurch gekennzeichnet, daß** sie weiterhin kosmetische Zubereitungsmittel umfaßt, wie zum Beispiel Duftstoffe, Färbemittel, antibakterielle Mittel, Insektenrepellents und Vitamine.

3. Zusammensetzung nach Anspruch 2, **dadurch gekennzeichnet, daß** das Färbemittel Karottenöl ist.

4. Zusammensetzung nach einem der Ansprüche 1 bis 3, **dadurch gekennzeichnet, daß** das UV-Strahlungsemissions-Filtermittel ausgewählt wird aus einer Gruppe, bestehend aus Ethylhexylmethoxycinnamat, Benzophenon-3, Aminobenzoesäure, Avobenzon, Phenylbenzimidazolsulfonsäure, Sulisobenzon, Cinoxato, Digaloyltrioleat, Diethanolaminmethoxycinnamat, Dioxybenzon, Ethyl-4-[bis(hydroxypropyl)]aminobenzoat, 2-Ethylhexyl-2-cyano-3,3-diphenylacrylat, Homosalat, Glycerylaminobenzoat, Methylantranylat, Octocrylen, Ethylhexylsalicilat, Padimat A, Padimat O und Tinosorb S.

5. Zusammensetzung nach einem der vorangehenden Ansprüche, **dadurch gekennzeichnet, daß** die Silica, welche im Gemisch von Silicas vorliegen, zirka 0,85 Gewichtsprozent bis zirka 7,5 Gewichtsprozent eines hydrophoberen Silicas und zirka 0,1 Gewichtsprozent bis zirka 7,5 Gewichtsprozent eines hydrophileren Silicas darstellen.

6. Zusammensetzung nach Anspruch 5, **dadurch gekennzeichnet, daß** die Silica, welche im Gemisch von Silicas vorliegen, zirka 0,9 Gewichtsprozent bis zirka 7,0 Gewichtsprozent eines hydrophoberen Silicas und zirka 0,15 Gewichtsprozent bis zirka 3,0 Gewichtsprozent eines hydrophileren Silicas darstellen.

7. Zusammensetzung nach Anspruch 6, **dadurch gekennzeichnet, daß** die Silica, welche im Gemisch von Silicas vorliegen, zirka 1,7 Gewichtsprozent bis zirka 5,3 Gewichtsprozent eines hydrophoberen Silicas und zirka 0,2 Gewichtsprozent bis zirka 1,7 Gewichtsprozent eines hydrophileren Silicas darstellen.

8. Zusammensetzung nach einem der Ansprüche 1 bis 7, **dadurch gekennzeichnet, daß** das Gemisch von Silicas, entweder separat oder als Kombination, ausgegastes Silica, amorphes Silica, geschmolzenes Silica, gesintertes Silica, kolloidales Silica, Silikatsalze, Silicatdimethylsililate und/oder Gemische derselben umfaßt.

9. Zusammensetzung nach einem der Ansprüche 1 bis 8, **dadurch gekennzeichnet, daß** der Träger ein Mineralöl ist.

10. Zusammensetzung nach einem der Ansprüche 1 bis 9, **dadurch gekennzeichnet, daß** die Bestandteile der Zubereitung in folgenden Mengen vorliegen:
a) zirka 0,1 Gewichtsprozent bis zirka 25 Gewichtsprozent eines UV-Strahlungs-Filtermittels;
b) zirka 1,0 Gewichtsprozent bis zirka 15,0 Gewichtsprozent eines Gemisches von Silicas;
c) zirka 0,001 Gewichtsprozent bis zirka 1 Gewichtsprozent Antioxidantien;
d) zirka 0,001 Gewichtsprozent bis zirka 5 Gewichtsprozent Vitamine; und
e) zirka 0,1 Gewichtsprozent bis zirka 1 Gewichtsprozent Duftstoffe.

11. Verwendung eines Gemisches von hydrophoben und hydrophilen Silicas, **dadurch gekennzeichnet, daß** sie bestimmt ist für die Zubereitung einer Zusammensetzung, wie in einem der Ansprüche 1 bis 10 definiert.

12. Verwendung nach Anspruch 11, **dadurch gekennzeichnet, daß** das Gemisch von Silicas die folgenden Prozentsätze aufweist:
i) zirka 0,85 Gewichtsprozent bis 7,5 Gewichtsprozent eines hydrophoberen Silicas; und
ii) zirka 0,1 Gewichtsprozent bis 7,5 Gewichtsprozent eines hydrophileren Silicas; besser:
i) 0,9 Gewichtsprozent eines hydrophoberen Silicas; und
ii) zirka 0,15 Gewichtsprozent bis 3,0 Gewichtsprozent eines hydrophileren Silicas; besser:
i) zirka 1,7 Gewichtsprozent bis 5,3 Gewichtsprozent eines hydrophoberen Silicas; und
ii) zirka 0,2 Gewichtsprozent bis 1,7 Gewichtsprozent eines hydrophileren Silicas.

13. Verwendung nach Anspruch 11 oder 12, **dadurch gekennzeichnet, daß** das Gemisch von Silicas, entweder separat oder als Kombination, ausgegastes Silica, amorphes Silica, geschmolzenes Silica, gesintertes Silica, kolloidales Silica, Silikatsalze, Silicatdimethylsililat und/oder Gemische derselben umfaßt.

14. Verwendung einer Zusammensetzung, wie in einem der Ansprüche 1 bis 10 definiert, **dadurch gekennzeichnet, daß** sie für die Zubereitung eines kosmetischen Produktes bestimmt ist.

15. Verwendung nach Anspruch 14, **dadurch gekennzeichnet, daß** das kosmetische Produkt ein Sonnenschutzmittel oder ein Bräunungs-Sonnenschutzmittel ist.

16. Verwendung nach Anspruch 14 oder 15, **dadurch gekennzeichnet, daß** das kosmetische Produkt ein Bräunungs-Sonnenschutzmittel in transparentem öligen Gel ist.

17. Verwendung einer Zusammensetzung, umfassend:
a) einen Träger;
b) ein UV-Strahlungs-Filtermittel;
c) ein hydrophobes Silica; und
d) ein hydrophiles Silica,
bei der Herstellung eines Medikaments für die Verhinderung von Hautkrankheiten, wobei das Medikament auf die Haut einer Person aufzutragen ist.

18. Verwendung nach Anspruch 17, **dadurch gekennzeichnet, daß** die Hautkrankheiten Störungen sind, die dadurch verursacht werden, daß die Haut einer Person Emissionen von UV-Strahlung ausgesetzt wird, und die ausgewählt werden aus einer Gruppe, bestehend aus Verbrennungen, Erythemen, Dermatosen, Hautentzündungen, Hautkrebsen und anderen.

## Revendications

1. Composition **caractérisée en ce qu'**elle renferme:
a) un véhicule ;
b) un agent pour filtrer les émissions de rayons UV;
c) une silice hydrophobe ; et
d) une silice hydrophile,
où ladite composition est sous la forme d'un liquide, d'un gel, d'un spray ou d'un applicateur à bille, et où la composition est à base d'huile.

2. Composition selon la revendication 1, **caractérisée en ce qu'**elle comprend en outre des agents de formulation tels que des agents de parfum, des agents colorants, des agents antibactériens, des agents pour repousser les insectes et des vitamines.

3. Composition selon la revendication 2, **caractérisée en ce que** l'agent colorant est de l'huile de carotte.

4. Composition selon l'une quelconque des revendications 1 à 3, **caractérisée en ce que** ledit agent pour filtrer les émissions de rayons UV est choisi dans un groupe constitué du méthoxycinamate d'éthylhexyle, du benzophénone-3, de l'acide aminobenzoïque, de l'avobenzone, de l'acide sulfonique de phénylbenzimidazol, du sulisobenzone, du cinoxato, du trioléate de digaloyle, du méthoxycinamate de diéthanolamine, du dioxybenzone, de l'éthyl 4-[bis(hydroxypropyl)]aminobenzoate, du 2-éthyl-hexyl 2-cyano-3,3-diphénylacrylate, de l'homosalate, de l'aminobenzoate de glycéryle, de l'anthranylate de méthyle, de l'octocrylène, du salicyate d'éthylhexyle, du padimate A, du padimate O et du Tinosorb S.

5. Composition selon l'une quelconque des revendications précédentes, **caractérisée en ce que** les silices présentes dans ledit mélange de silices représentent d'environ 0,85 % à environ 7,5 % en poids d'une silice plus hydrophobe et d'environ 0,1 % à environ 7,5 % en poids d'une silice plus hydrophile.

6. Composition selon la revendication 5, **caractérisée en ce que** les silices présentes dans ledit mélange de silices représentent d'environ 0,9 % à environ 7,0 % en poids d'une silice plus hydrophobe et d'environ 0,15 % à environ 3,0 % en poids d'une silice plus hydrophile.

7. Composition selon la revendication 6, **caractérisée en ce que** les silices présentes dans ledit mélange de silices représentent d'environ 1,7 à environ 5,3 % en poids d'une silice plus hydrophobe et d'environ 0,2 à environ 1,7 % en poids d'une silice plus hydrophile.

8. Composition selon l'une quelconque des revendications 1 à 7, **caractérisée en ce que** ledit mélange de silices renferme, soit séparément soit de manière combinée, de la silice fumigée, de la silice amorphe, de la silice fondue, de la silice vitreuse, de la silice colloïdale, des sels de silicate, du sililate diméthylique de silice et/ou des mélanges de ceux-ci.

9. Composition selon l'une quelconque des revendications 1 à 8, **caractérisée en ce que** ledit véhicule est une huile minérale.

10. Composition selon l'une quelconque des revendications 1 à 9, **caractérisée en ce que** les composants de la formulation sont présents dans les quantités suivantes :
a) d'environ 0,1 % à environ 25 % en poids d'agents pour filtrer les émissions de rayons UV ;
b) d'environ 1,0 % à environ 15,0 % en poids d'un mélange de silices ;
c) d'environ 0,001 à 1 % en poids d'antioxydants;
d) d'environ 0,001 à environ 5 % en poids de vitamines ; et
e) d'environ 0,1 % à environ 1 % en poids de parfums.

11. Utilisation d'un mélange de silices hydrophobes et hydrophiles, **caractérisée en ce que** le mélange est destiné à la préparation d'une composition telle que définie dans l'une quelconque des revendications 1 à 10.

12. Utilisation selon la revendication 11, **caractérisée en ce que** ledit mélange de silices a les proportions suivantes :
i) d'environ 0,85 % à 7,5 % en poids d'une silice plus hydrophobe ; et
ii) d'environ 0,1 % à 7,5 % en poids d'une silice plus hydrophile ; mieux
i) 0,9 % en poids d'une silice plus hydrophobe ; et
ii) d'environ 0,15 % à 3,0 % en poids d'une silice plus hydrophile ; et mieux encore
i) d'environ 1,7 à 5,3 % en poids d'une silice plus hydrophobe; et
ii) d'environ 0,2 % à 1,7 % en poids d'une silice plus hydrophile.

13. Utilisation selon la revendication 11 ou 12, **caractérisée en ce que** ledit mélange de silices renferme, soit séparément soit de manière combinée, de la silice fumigée, de la silice amorphe, de la silice fondue, de la silice vitreuse, de la silice colloïdale, des sels de silicate, du sililate diméthylique de silice et/ou des mélanges de ceux-ci.

14. Utilisation d'une composition selon l'une quelconque des revendications 1 à 10, **caractérisée en ce que** la composition est destinée à la préparation d'un produit cosmétique.

15. Utilisation selon la revendication 14, **caractérisée en ce que** ledit produit cosmétique est un écran solaire ou un écran solaire bronzant.

16. Utilisation selon la revendication 14 ou 15, **caractérisée en ce que** ledit produit cosmétique est un écran solaire bronzant sous la forme d'un gel huileux transparent.

17. Utilisation d'une composition renfermant:
a) un véhicule ;
b) un agent pour filtrer les émissions de rayons UV ;
c) une silice hydrophobe ; et
d) une silice hydrophile
pour la fabrication d'un médicament pour prévenir les maladies de la peau, dans lequel le médicament est destiné à être appliqué à la peau d'une personne.

18. Utilisation selon la revendication 17, **caractérisée en ce que** lesdites maladies de la peau sont les désordres causés par l'exposition de la peau d'une personne à des émissions de rayons UV, qui sont choisis dans un groupe constitué des brûlures, de l'érythème, des dermatoses, des dermatites, des cancers de la peau et autres.
